# EUROPEAN PATENT APPLICATION

(11) **EP 2 551 276 A1**
(43) Date of publication of application: **30.01.2013**
(21) Application number: 11175472.7
(22) Date of filing: 26.07.2011
(51) Int. Cl.: C07K 1/20

(54) **Method for purification of immunoproteasomes**

(71) Applicant: ARES TRADING S.A., 1170 Aubonne (CH)
(72) Inventor: Dechavanne, Vincent, 74160 Beaumont (FR); Antonsson, Bruno, 01200 Billiant (FR)
(74) Representative: Merck Serono SA - Geneva Intellectual Property

(57) **Abstract**

The present invention provides a method for the purification of immunoproteasomes comprising subjecting an immunoproteasome solution to hydrophobic interaction chromatography, wherein the hydrophobic interaction chromatography is performed with a resin comprising butyl residues.

## Description

### FIELD OF THE INVENTION

The present invention is in the field of protein purification. More specifically, it relates to a method for the purification of immunoproteasomes based on hydrophobic interaction chromatography.

### BACKGROUND OF THE INVENTION

Proteasomes are highly conserved macromolecular structures which are responsible for the majority of non-lysosomal protein degradation (Coux O et al. 1996) and are widely distributed in various eukaryotic cells and tissues ranging from human to yeast (Tanaka K et al, 1988). They are found in the cytoplasm and nucleus of eukaryotic tissues and consist of at least 14 non-identical subunits with molecular masses ranging from approximately 20 to 32kDa.

The 26S proteasome complex (2000-kDa) is composed of two subparticles, the 20S core protease (CP) that compartmentalizes the protease active sites and the 19S regulatory particle that recognizes and translocates appropriate substrates into the CP lumen for breakdown (Voges D, 1999, Demartino GN, Gillette TG, 2007). The 20S catalytic core is a 700-kDa barrel-shaped particle composed of 28 subunits assembled in four stacked seven-membered rings (Groll M. et al. 1997). The outer rings contain seven different non catalytic α type subunits, and the inner rings contain seven different β type subunits. The two outer α subunit rings participate in proteasome assembly (Zwickl P., 1994), control substrate entry (Osmulski PA and Gaczynska M., 2000), and associate with regulatory particles such as PA28 and PA700 (Kania MA. et al., 1996). The active proteolytic sites are located in the β subunits that constitute the two inner rings of the particle, three of which have catalytic activity located on the interior surface of the rings (β1, β2 and β5) with caspase-like, trypsin-like and chymotrypsin-like activities, respectively (Groll M et al., 1997). The 20S proteasome function through a new type of proteolytic mechanism involving a threonine active site. The 19S particle can be dissociated into two subcomplexes called the base and the lid (Glickman MH, et al, 1998). The lid subcomplex covers the base and is thought to be involved in the recognition of ubiquitin and ubiquitin chain processing of substrates before their translocation and degradation. The subunit composition of the 20S proteasome can vary and is subject to precise regulation. In addition to core subunits, a number of other factors associate substoichiometrically with the 26S proteasome.

Mammalian 20S proteasomes have been classified into two groups: 1) the constitutive proteasome (cP), constitutively expressed in all eukaryotic cells, with the three catalytic β subunits: β1, β2 and β5 and 2) the immunoproteasome (iP) formed upon stimulation of cells by γ interferon. The three constitutive β subunits are replaced in the immunoproteasome by distinct, newly synthesized ones, subunits β1i [also named low molecular weight protein 2 (LMP2), β2i [multicatalytic endopeptidase complex-like 1 (MECL1)] and β5i (LMP7) (Brown MG and Monaco JJ., 1993; Akiyama K, et al, 1994), which alter the activities of the proteasome so as to favor antigen presentation (Gaczynska M, et al, 1993; Driscoll J, et al. 1993).

Intermediate immunoproteasome is a hybrid proteasome composed of IFN-γ-inducible β subunits and catalytic β subunits.

It has been shown that proteasomes are responsible for generation of cytosolic peptides of 7-13 amino acids in length, which are presented on cell surfaces in association with major histocompatibility complex class I (MHC-1) molecules (Monaco JJ, Nandi D., 1995; Nandi D, et al. 1998). Aside from the physiological difference between standard and immunoproteasome activities, the proteasome catalytic activity may be affected by various environmental factors such as oxidative stress, pathological states such as cancers or neurological disorders, or pharmacological agents (Shah SA, et al. 2001). The impact of structural modifications, and more particularly post-translational modifications that affect either the protein to be degraded or the proteasome subunits, may lead to altered or even inhibited proteolytic functions. The level of the 26S proteasome is particularly upregulated in tumor cells.

It is therefore possible to develop specific inhibitors targeting proteasome mediated degradation for cancer treatment. Bortezomib (PS-341, Velcade®) developed by Millenium Pharmaceuticals Inc., which inhibits cytokine production in vitro and inflammation in vivo, was approved for myeloma therapy in the United states in 2006, raising expectations that the proteasome would be a novel target for cancer treatment (Sánchez-Serrano I., 2006). Another small molecule: Salinosporamide A (NPI-0052), isolated from Actinomycete (Feling RH, 2003), was found to be more effective than bortezomid and is undergoing clinical trials as a candidate drug for chemical therapy.

Selective inhibition of the LMP7 subunit of the immunoproteasome was shown to block inflammatory cytokine secretion in human PBMC, thus making the immunoproteasome an interesting target to fight against autoimmune diseases. However, most well characterized proteasome inhibitors mediate equivalent inhibition of both proteasome chymotrypsin-like activities (β5 and LMP7) and have considerable toxicities that probably limit their clinical utility in chronic inflammatory diseases such as rheumatoid arthritis (Bross PF et al, 2004). Pure preparations of immunoproteasome are thus necessary to perform crystallization studies and to develop specific inhibitors in support of drug development programs.

Several purification strategies are frequently encountered to purify proteasomes whatever the cell line used due to the highly conserve structure of the proteasome from yeast to human. Conventional purification of the 26S proteasome involves multiple chromatographic steps and usually takes several days. Gel filtration chromatography or ultracentrifugation, since proteasomes are much larger than most other cellular proteins are frequently used, in combination with other conventional chromatography techniques, like anion exchange chromatography coupled to hydrophobic interaction chromatography or ammonium sulfate precipitation (Tenzer S, Schild H., 2005, Eleuteri AM, et al 2000, Groettrup M et al, 1995, Orlowski M, Michaud C., 1989, Sacchetta P, et al. 1990, Dahlmann et al., 2000). The combination of such chromatography techniques is able to provide 95 to 99% of purity. Chapiro and al (2006), described a simple method to purify proteasome from human erythrocytes by affinity chromatography with the monoclonal antibody MCP21 (European Collection of Cell Cultures) directed against the human α2 subunit. Others chromatography techniques like Hydroxyapatite (Bauer MW, 1997) and heparine sepharose were also combined with success (Saeki Y et al, 2005). Based on these strategies, it was possible to reach the expected purity of proteasome. Unfortunately, it is more difficult to separate the residual constitutive proteasome from the immunoproteasome after stimulation of cells by γ interferon. Purified immunoproteasome (iP) preparations that are commercially available (e.g. from Boston Biochem) also show a certain level of cross contamination by the constitutive proteasome.

An ongoing need remains for a new simple and efficient method for separating the immunoproteasome from the constitutive proteasome and the intermediate immunoproteasome, which would permit to produce pure and specific reagents to provide new inhibitors of the LMP7 subunit.

### SUMMARY OF THE INVENTION

It is an object of the invention to provide a new method for purifying immunoproteasomes.

The invention provides a method for the purification of immunoproteasomes comprising subjecting an immunoproteasome solution to hydrophobic interaction chromatography, wherein the hydrophobic interaction chromatography is performed with a resin comprising butyl residues.

In a preferred embodiment, the purification method of the invention comprises the steps of:
a. subjecting an immunoproteasome solution to anion exchange chromatography to produce a first eluate;
b. subjecting the eluate of step (a) to mixed mode chromatography;
c. subjecting the eluate of step (b) to hydrophobic interaction chromatography wherein the hydrophobic interaction chromatography is performed with a resin comprising butyl residues; and
d. collecting the fraction comprising the immunoproteasome from the eluate of step (c).

### DETAILED DESCRIPTION OF THE INVENTION

The proteasome is a multicatalytic protease complex present in all eukaryotic cells which plays a critical role in regulating essential cellular processes (see also the detailed description in the "Background of the invention" section). The proteasome subcomponents are often referred to by their Svedberg sedimentation coefficient (denoted S). The most common form of the proteasome is known as the 26S proteasome, which is about 2000 kilodaltons (kDa) in molecular mass and contains one 20S core particle structure and two 19S regulatory caps. The 20S proteasome has three catalytic β subunits: β1, β2 and β5 and is constitutively expressed in all eukaryotic cells (the "constitutive proteasome" or "cP"). In response to exposure to pro-inflammatory signals such as cytokines, in particular, interferon gamma alternative β subunits denoted β1i (LMP2), β2i (MECL1) and β5i (LMP7) are expressed and the proteasome assembled with these subunits is known as the "immunoproteasome(s)" (iP). "intermediate immunoproteasome(s)" or "hybrid proteasome(s)", as used herein, refers to a hybrid proteasome composed of a mix of IFN-γ-inducible β subunits (β1i, β2i and β5i) and catalytic β subunits (β1, β2 and β5).

The increase of proteosomal activity has been linked to inflammatory and autoimmune diseases. Selective inhibition of subunits of the immunoproteasome such as LMP7 (β5i) was shown to block inflammatory cytokine secretion in human PBMC, thus making the immunoproteasome an interesting target to fight against autoimmune diseases. Pure preparations of immunoproteasomes is a key step in the identification of specific inhibitors of immunoproteasomes.

Immunoproteasome is present in relatively high quantities in different immune cells, however, the cells also contain other proteasome complexes such as the constitutive proteasome or intermediate immunoproteasome. Thus, preparations of immunoproteasome frequently contain considerable contamination by constitutive proteasomes and/or intermediate immunoproteasomes.

"Proteasome complexes", "proteasome fractions", "proteasome species" or "proteasome(s)", as used herein, refer to a variety of proteasomes including immunoproteasomes, constitutive proteasomes and intermediate immunoproteasomes.

The present invention presents an outstanding method of purification enabling the separation of the immunoproteasome from other proteasome complexes, such as the constitutive proteasome and/or intermediate immunoproteasome, based on hydrophobic interactions chromatography. This method allows the production of pure immunoproteasomes reducing thereby the cross-contamination with other proteasomes complexes. In addition, this method is suitable to produce large quantities of immunoproteasomes.

The invention relates to the use of the hydrophobic interaction chromatography resin comprising butyl residues for the purification of immunoproteasomes. The invention provides a method for the purification of immunoproteasomes comprising subjecting a solution containing immunoproteasomes to hydrophobic interaction chromatography (HIC), wherein the hydrophobic interaction chromatography is performed with a resin comprising butyl residues.

The fraction comprising the purified immunoproteasomes is collected from the eluate of the HIC step.

The solution containing the immunoproteasomes, hereafter the "immunoproteasome solution", may be any composition or preparation of a fluid derived from a cell culture, such as e.g. a crude cell extract or cell culture harvest. It may also be a fluid derived from another purification step, such as e.g. the eluate or flow-through from a capture step or any other suitable purification step preceding the hydrophobic interaction chromatography step.

The hydrophobic interaction chromatography step according to the method of the present invention may be used in a purification method having one or more additional steps. The additional steps may precede or follow the hydrophobic interaction chromatography step. The additional steps can be selected from anion exchange chromatography (AEX), mixed mode chromatography or size size-exclusion chromatography (SEC). Preferably, the anion exchange chromatography and/or mixed mode chromatography precede the HIC step whereas the size-exclusion chromatography follows it. Preferably the steps are carried out in the following order:
a. Anion exchange chromatography;
b. Mixed mode chromatography; and
c. Hydrophobic interaction chromatography.

In a preferred embodiment, the invention relates to a method for the purification of immunoproteasomes from an immunoproteasome solution comprising the steps of :
a. subjecting an immunoproteasome solution to anion exchange chromatography to produce a first eluate;
b. subjecting the eluate of step (a) to mixed mode chromatography;
c. subjecting the eluate of step (b) to hydrophobic interaction chromatography wherein the hydrophobic interaction chromatography is performed with a resin comprising butyl residues; and
d. collecting the fraction comprising the immunoproteasome from the eluate of step (c).

The elution of the proteasome complexes from the hydrophobic interaction chromatography column is performed with a gradient, preferably a decreasing salt gradient. The salts are usually detected by monitoring the conductivity of the buffer.

The elution of the bound fractions is monitored by their UV absorbance at A_{280 nm}. The proteasome fractions corresponding to the immunoproteasomes, the constitutive proteasomes and intermediates immunoproteasomes, elute as distinctive peaks (main peaks) from the HIC column and are collected separately. When a decreasing salt gradient is used, three main peaks elute from the HIC column as follows: the first one, eluting at an average conductivity of 118mS/cm, correspond to the immunoproteasome fraction; a second peak eluting at 112mS/cm correspond to the intermediate immunoproteasome and finally a third one eluting at 99mS/cm correspond to the constitutive proteasome. The fraction comprising the purified immunoproteasome in therefore collected from the eluate of the HIC step.

In a further preferred embodiment, the eluate fractions of the hydrophobic interaction chromatography are subjected to size-exclusion chromatography as a polishing step (step e). Preferably, the eluate fraction comprising the immunoproteasomes is subjected to size-exclusion chromatography.

After any of the steps of chromatography, the eluate can be subjected to an intermediate step of dialysis, diafiltration or ultrafiltration prior to subjecting it to a further chromatography step. In a preferred embodiment, the eluate of the mixed mode chromatography is subjected to dialysis prior to loading on the hydrophobic interaction chromatography column.

The immunoproteasome solution to be purified can be obtained by culturing eukaryotic cell lines expressing constitutive proteasome and stimulating such cells with gamma interferon (IFN-γ), preferably recombinant gamma interferon (rIFN-γ). The cell lines may be yeast or mammalian cells. The mammalian cell line is preferably a human cell line expressing human constitutive proteasome that is stimulated with recombinant human gamma interferon (rIFN-γ). Human epithelial cell lines A549 and Burkitt's lymphoma cell line Ramos are examples of human cell lines expressing constitutive proteasome.

The immunoproteasome solution may further contain other proteasome complexes such as constitutive proteasomes and/or intermediate immunoproteasomes.

In a preferred embodiment, the immunoproteasome is purified from an immunoproteasome solution comprising constitutive proteasomes and intermediate immunoproteasomes.

The hydrophobic interaction chromatography purification step of the present invention as well as the anion exchange chromatography, mixed mode chromatography and size-exclusion chromatography are in the following outlined more in detail.

### Anion exchange chromatography (AEX)

I n a preferred embodiment, the method of the invention involves a step of anion exchange chromatography. Preferably, the anion exchange resin comprises diethylaminoethyl (DEAE).

A preferred resin is the weak anion exchanger Toyopearl^{®} DEAE-650M (obtainable from TosoHaas). Its preferred technical features are the following:

| | |
|---|---|
| Product Name | Toyopearl® DEAE-650 (weak anion exchange resin) |
| Structure | HW - 65 - O-CH2-CH2-N-(C2H5)2 |
| Formulation | Particulate resin (spherical), available in particle sizes (mean diameter): |
| | 65 µm = "M" grade |
| | Supplied as slurry in 20 % ethanol (v/v). |
| Physicochemical Specifications | |
| Appearance | White resin slurry which settles upon standing |
| Particle Size Distribution (>80 % within range) | 40 - 90 µm for DEAE-650M |
| Exclusion Limit | 5 x10⁶ Dalton (globular protein) |
| Mean Pore Diameter | 1000 A |
| Pressure drop across the column | max. 7 bar (recommended) |
| Operating Linear Flowrate | normally 10 - 600 cm/hour (depending on particle size) |
| Ion Exchange Capacity | DEAE-650S,M 0.1 ± 0.02 meq/mL |
| Protein Adsorption Capacity | 30 ± 5 mg/mL (Bovine Serum Albumin) |
| Bacterial Count | ≤ 100 CFU/mL |
| Endotoxin Concentration | ≤ 10.0 endotoxin units/mL |
| Pack sizes and type | 50 mL, 100 mL, 250 mL, 1 L, 5 L, 50 L Packed in virgin Polyethylene container with Polypropylene cap. |
| Resin Volume per Container | The indicated volume is the settled resin volume. |
| Shipping Solvent | 72 % (v/v) slurry in 20 % (v/v) ethanol |
| Long Term Storage Conditions | 20 % (v/v) ethanol |
| Cleaning in Place / Sanitization | 0.5 M NaOH or 0.1 M HCl |
| Foreign Substance | not observed, no visual evidence |
| Eluable Matter | < 0.2 % |
| Shelf Life Stability | Minimum 5 years |

Examples of alternate resins include: DEAE ceramic HyperD resin from PALL corporation, DEAE sepharose Fast Flow from GE Healthcare or Fractogel® EMD DEAE (M).

The anion exchange column is preferably equilibrated with an appropriate equilibration buffer. The equilibration buffer is preferably the binding buffer.

An appropriate binding buffer is e.g. a buffer containing 1 mM DTT, 25 mM NaCl, 10 % glycerol, 20 mM Triethanolamine pH 7.7. Suitable pH values for the equilibration, washing and loading buffer range from about 6.0 to about 8.0, preferably from about 6.8 to about 8, e.g. at about 7.7.

In a preferred embodiment, the crude cell extracts are loaded directly on the anion exchange column. After loading, the column is washed with an appropriate wash buffer then elution is performed. Preferably, two elution steps may be performed. A first elution step with 12% of elution buffer (1 mM DTT, 10 % glycerol, 500 mM NaCl, 20 mM, triethanolamine pH 7.7) is implemented to remove contaminants. Subsequently, a gradient from 12 to 60% of elution buffer is applied. The eluate should contain the different proteasome species.

### Mixed mode chromatography

In a preferred embodiment the method of the invention involves Mixed mode chromatography. Preferably, the mixed mode chromatography uses ceramic hydroxyapatite as adsorbant; it is most preferably ceramic hydroxyapatite type II. A most preferred resin CHT Ceramic Hydroxyapatite Type II obtainable from Bio-Rad. Its technical features are the following:

| | |
|---|---|
| Functional groups | Ca²⁺, PO₄³⁻, OH |
| Particle sizes available | 20, 40 and 80 µm (nominal) |
| Recommended linear flow rate | 50-1,000 cm/hr |
| Operating pH range | 6.5-14 |
| Chemical compatibility (>24 hr) | 1 M NaOH, 8 M urea, 8 M guanidine-HCl, |
| | ethanol, methanol, 100% acetonitrile |
| Regeneration | 0.4-1.0 M phosphate buffer, 0.5 M sodium phosphate pH 7 |
| | 1.0 M trisodium phosphate pH 11-12 |
| Sanitization | 1-2 N NaOH |
| Autoclavability (121 µC, 20 min) | Yes |
| Packing density (g/ml packed bed) | 0.63 g/ml |
| Dynamic binding capacity | > 12.5 mg lysozyme/g |
| Typical IgG binding capacities at 500 cm/hr | 15-25 mg/ml |
| Nominal pore diameter | 800-1,000 A |
| Maximum operating pressure | 100 bar (1,500 psi) |

It is understood that the method may be performed with alternate resins, having similar characteristics. Examples of alternative resins include: HA Ultrogel® Hydroxyapatite from Pall corporation, TSK gel HA-1000 from TosoHass or Bio-Gel® HTP Hydroxyapatite from Bio-Rad.

The mixed mode column is preferably equilibrated with an appropriate equilibration buffer. The equilibration buffer preferably comprises potassium-phosphate. Optionally, the sodium phosphate buffer is at a concentration between about 1 mM and about 50 mM, in another embodiment at a concentration between about 10 mM and 30 mM. Preferably, the equilibration takes place at a pH of about 6.8.

An appropriate binding buffer is e.g. a buffer 1 mM DTT, 10 % glycerol, 10 mM potassium-phosphate pH 6.8. Suitable pH values for the equilibration/washing/loading buffer range from about 5.0 to about 8.0, preferably from about 6.5 to about 7.5, e.g. at about 6.8.

After loading, the column is washed with an appropriate wash buffer then elution is performed. Adsorbed proteins are eluted with a linear gradient of potassium phosphate at pH 6.8 from 0 to 300 mM.

### Hydrophobic interaction chromatography (HIC)

The method of the invention involves a step of hydrophobic interaction chromatography, wherein the hydrophobic interaction chromatography is carried out using a resin comprising butyl residues.

A preferred resin is Butyl HP sepharose (obtainable from GE-Healthcare Lifesciences, supplier's reference: 17-5432-02). Its preferred technical features are the following:

| Butyl Sepharose High Performance Main characteristics | |
|---|---|
| Functional group | Butyl -O-(CH2)3-CH3 |
| Matrix | Highly cross-linked agarose |
| Average particle size | 34 µm |
| Exclusion limit (Mr) | 4 × 10⁶ (globular proteins) |
| Ligand concentration (µmol/ml medium) | 50 µmol/ml medium |
| Binding capacity | 38 mg β-lactoglobulin/ml medium |
| Rec. linear flow rate | Up to 150 cm/h |
| Chemical stability | 1 M sodium hydroxide |
| | 1 M acetic acid |
| | 8 M urea |
| | 6 M guanidine hydrochloride |
| | 30% acetonitrile |
| | 70% ethanol |
| | 3 M (NH₄)₂SO₄ |
| | 1 mM HCl |
| | 30% isopropanol |
| | 2% SDS |

Alternate butyl resins, having similar characteristics are for example Butyl-650M from Toyopearl^{®}(the size of particles are ranging from 40 to 90µm) or Fractogel TSK Butyl 650 from Merck. Nevertheless, the resolution of the separation will depend on the size of the beads especially when using small beads.

The hydrophobic interaction chromatography (HIC) column according to the present invention is preferably equilibrated with an appropriate equilibration buffer. The equilibration buffer may be the same as the binding buffer.

An appropriate binding buffer is e.g. a buffer comprising ammonium sulfate at 1.7 M (NH₄)₂SO₄**-** Suitable pH values for the equilibration/washing/loading buffer range from about 5.0 to about 8.0, preferably from about 6.5 to about 7.5, e.g. at about 6.7. Other anti-chaotropic salts than sodium sulfate may be used such as for example sodium phosphate (Na₂SO₄) ranging from 1 to 2 M. Alternatively, sodium chloride (NaCl) can also be used at a concentration ranging from 2 to 4 M.

Prior to loading on the hydrophobic interaction chromatography column, the eluate from a preceding step is preferably dialyzed or diafiltered into an appropriate loading buffer containing ammonium sulfate or sodium chloride. In case mixed mode chromatography is carried out before the HIC step, the eluate from the mixed mode chromatography column is preferably dialyzed against ammonium sulfate, diluted into an appropriate loading buffer and loaded on the HIC column. Most preferably, the dialysis of the eluate from the mixed mode chromatography is performed against ammonium sulfate at about pH 6.7

In case anion exchange chromatography (AEX) is carried out before the HIC step, the eluate is preferably diafiltered or diluted against a buffer containing 1.7M of ammonium sulfate and directly loaded onto the HIC column.

After loading, the HIC column is washed with the equilibration buffer (1m DTT, 1.7M (NH₄)₂SO₄, 50 mM Tris-HCI pH 6.7).

The elution from the HIC column is performed with gradient elution. When gradient elution is performed, the bound fractions are eluted from the HIC resin with a decreasing salt gradient buffer consisting of about 1.7 to 0M (NH₄)₂SO₄, about 2 to 0M Na₂SO₄ or 4 to 0M NaCl.

The eluate of the HIC, comprising the various proteasome fractions is collected. The proteasome complexes elute as distinctive peaks and the fractions are collected separately. A first peak eluting at an average conductivity of 118mS/cm correspond to the immunoproteasome fraction, then a second peak at 112mS/cm correspond to the intermediate immunoproteasome fraction and finally a third one at 99mS/cm correspond to the constitutive proteasome fraction.

### Size exclusion chromatography

In a further embodiment, the method of the invention involves subjecting the fractions eluted from the HIC column to size-exclusion chromatography. This step is preferably performed using a gel filtration matrix selected from the group consisting of e.g. Superdex 200, Superose 6, superpose 12 from GE Healthcare, Ultrogel® AcA 22 or 34 from TosoHass.

A preferred resin is Hiload 16/60 superdex 200 prep grade (obtainable GE-Healthcare Lifesciences).

The volume of the resin, the length and diameter of the column to be used, as well as the dynamic capacity and flow-rate to be used in the various purification steps of the method of the invention depend on several parameters such as the volume of fluid to be treated, concentration of protein in the fluid to be subjected to the process of the invention, etc. Determination of these parameters for each step is well within the average skills of the person skilled in the art.

The examples herebelow are provided to further illustrate the method of the invention.

### BRIEF DESCRIPTION OF THE DRAWINGS

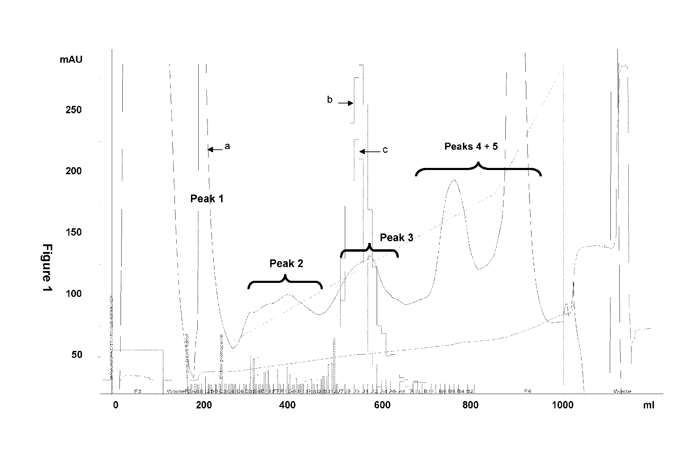

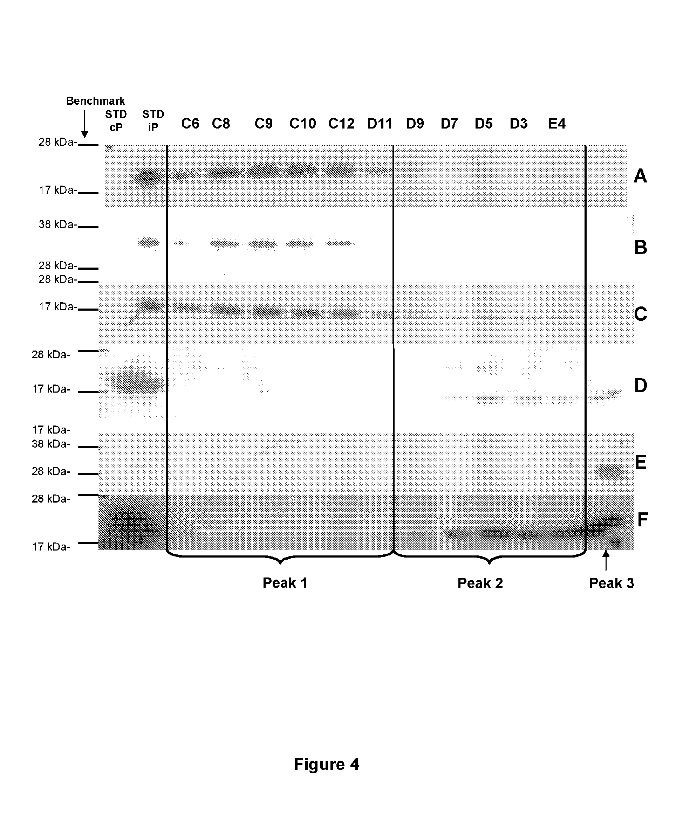

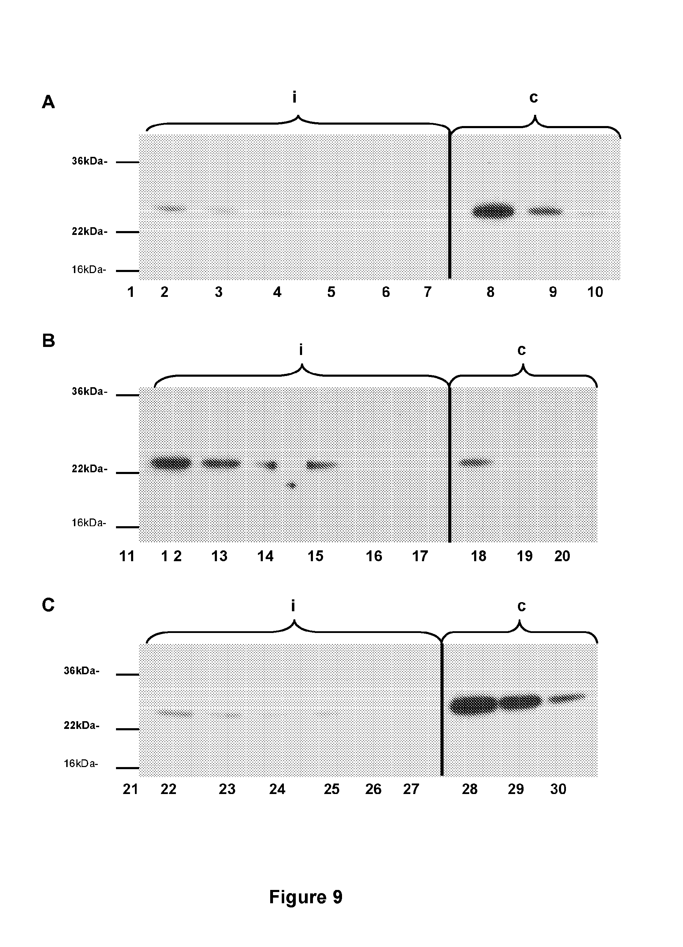

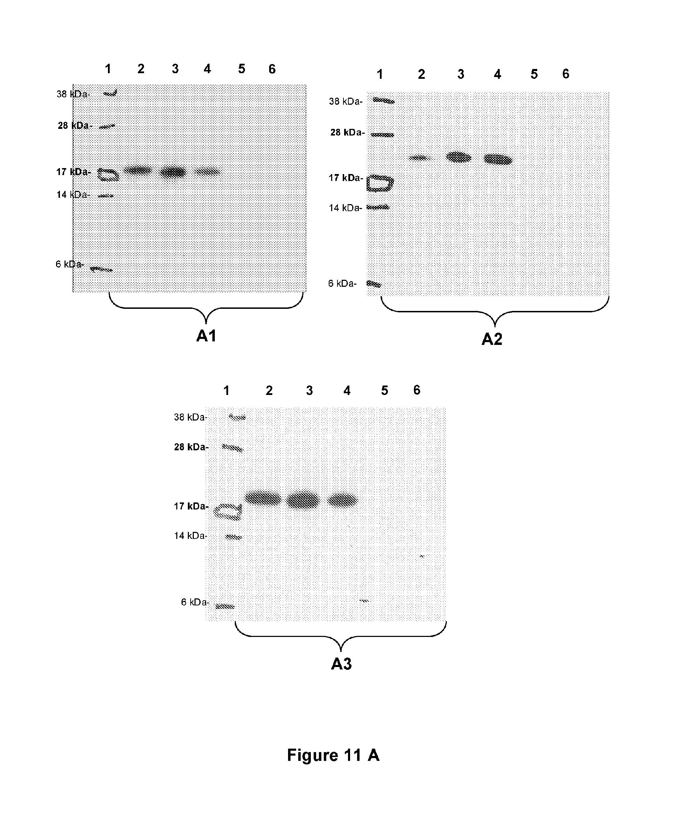

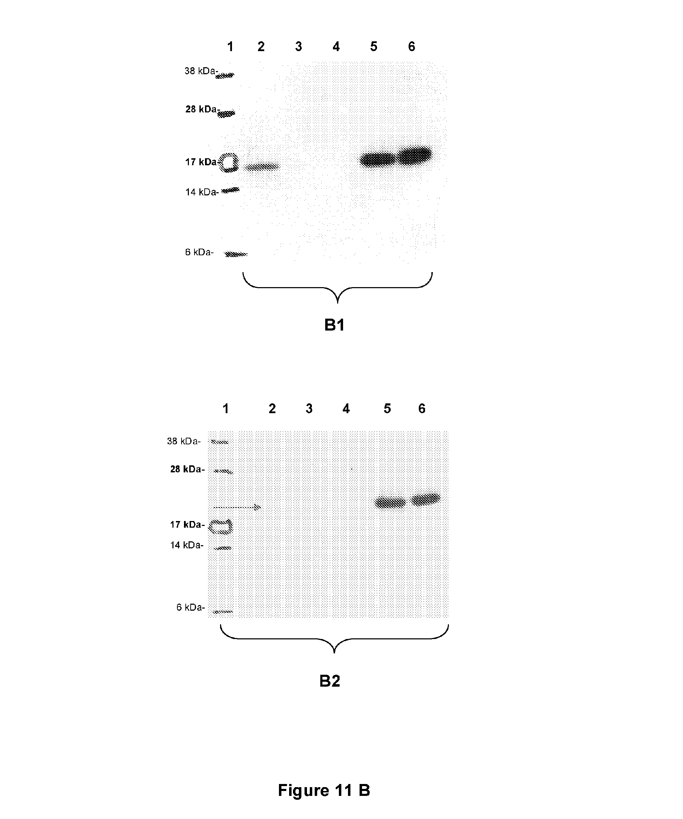

**Figure 1****.** DEAE-650M column chromatography. The crude extract harvested from the A549 cell line culture pre-activated with IFNγ was filtered on 0.2 µm. The filtrate was loaded onto a DEAE-650M column. Bound proteins were eluted with a gradient of NaCl. Each fraction was tested for its capacity to hydrolyze both peptides: LLVY-AMC (chymotrypsin-like activity corresponding to the bars designated "b") and LLE-βNa (caspase-like activity corresponding to the bars designated "c"). Major activities eluted between 0.17 and 0.25M NaCl coincident with as single peak of protein (peak 3). Fractions containing the highest activities were pooled.

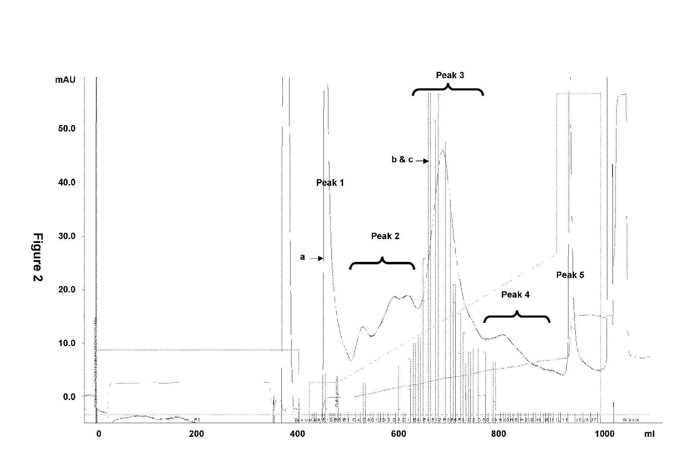

**Figure 2****.** HAE ceramic type II column chromatography from DEAE-650M (a). Pooled DEAE-650M fractions were directly loaded onto a Hydroxyapatite column equilibrated in 10 mM Na-phosphate, 1 mM DTT, 10% glycerol pH 6.8. Bound proteins were eluted with a sodium phosphate gradient (10-300 mM) and each fraction was tested for its capacity to hydrolyze both peptides: LLVY-AMC (chymotrypsin-like activity) and LLE-βNa (caspase-like activity). Major activities were recovered in the peak 3 which was eluted between 70 and 106 mM of Na-phosphate. Fractions containing the highest activities were pooled. The bars designated b and c correspond to chymotrypsin-like activity and caspase-like activity that are superimposed.

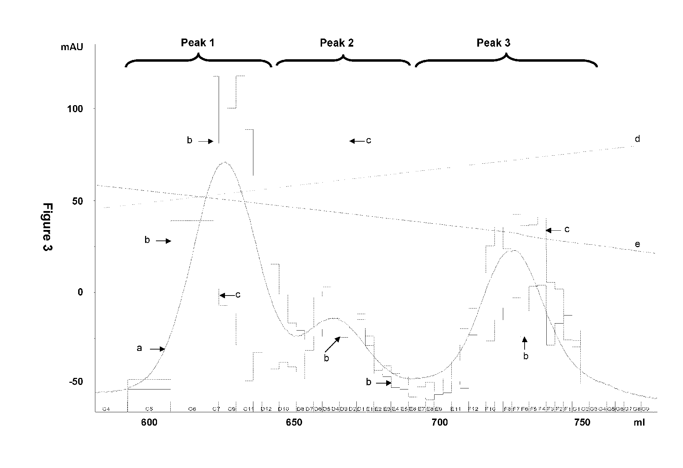

**Figure 3****.** Butyl HP sepharose column chromatography of proteasome from HAE chromatography (a). The pooled HAE fractions were dialyzed overnight in a buffer containing 1.7M (NH₄)₂SO₄ and then centrifuged at 10.000g for 30 minutes. The supernatant was recovered and loaded onto a Butyl HP sepharose column. Bound proteins were eluted with a decreasing gradient of (NH₄)₂SO₄. Each fraction was tested for its capacity to hydrolyze both peptides: LLVY-AMC (chymotrypsin-like activity corresponding to the bars designated "b") and LLE-βNa (caspase-like activity corresponding to the bars designated "c"). The three main peaks were pooled separately and each peak was further characterized by western blot in order to identify confidently their subunit contents.
   Peak 1: Immunoproteasome. Conductivity at 118 mS/cm
   Peak 2: Intermediate immonoproteasome. Conductivity at 112 mS/cm
   Peak 3: Constitutive proteasome ((β1+β2+β5). Conductivity at 99 mS/cm
   d: gradient
   e: conductivity in mS/cm
**Figure 4****.** Identification of constitutive proteasome and immunoproteasome within the Butyl HP chromatography fractions by Western blot with antibodies directed specifically against constitutive proteasome subunits or immunoproteasome subunits.
   Row A: identification of the β1i subunit for the immunoproteasome with a monoclonal antibody from Enzo life Science (clone LMP2).
   Row B: identification of the β2i subunit for the immunoproteasome with a monoclonal antibody from Santa Cruz (clone MECL1 C2).
   Row C: identification of the β5i subunit for the immunoproteasome with a monoclonal antibody from Enzo life Science (clone LMP7-1).
   Row D: identification of the β1 subunit for the constitutive proteasome with a monoclonal antibody from Enzo life Science (clone MCP 421).
   Row E: identification of the β2 subunit for the constitutive proteasome with a rabbit polyclonal antibody from Boston Biochem (AP 123).
   Row F: identification of the β5 subunit for the constitutive proteasome with a rabbit polyclonal antibody from UpState laboratory (09-278).
   Lanes C6, C8, C9, C10, C12 and D11: fractions from elution peak 1 (immunoproteasome). Lanes D9, D7, D5, D3, E4: fractions from elution peak 2 (intermediate immonoproteasome).
   Last lane: fraction from elution peak 3 (constitutive proteasome)
   STD cP: standard for constitutive proteasome.
   STD iP: standard for the immunoproteasome.

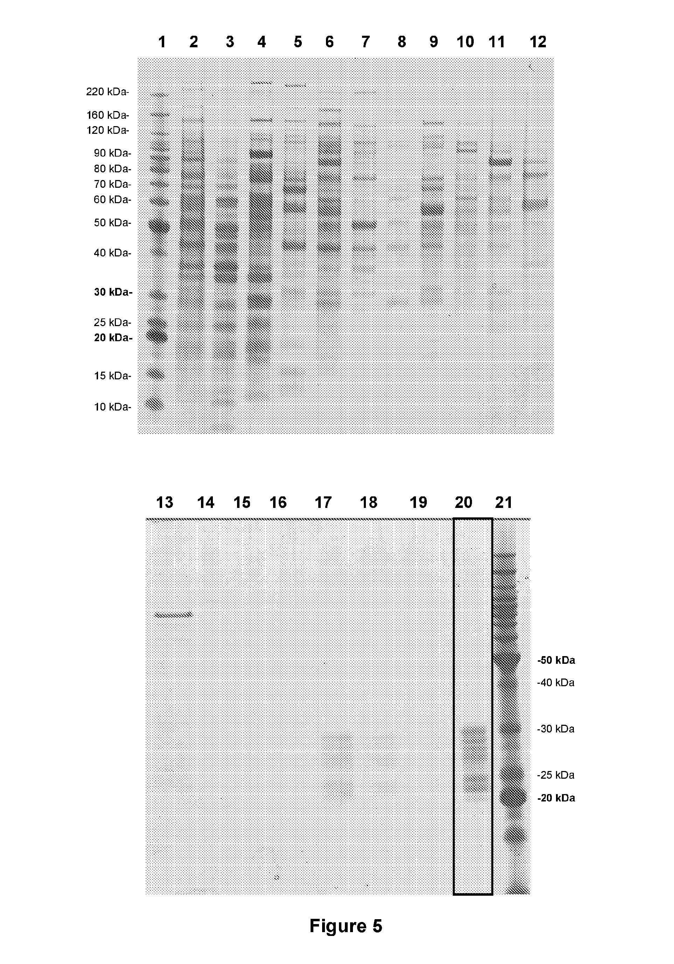

**Figure 5**. Control in-process by SDS-PAGE in reducing conditions.
   Lane 1: Benchmark protein ladder.
   Lane 2: Crude extract filtered on 0.2µm.
   Lane 3: Flow through fraction from DEAE-650M column.
   Lane 4: Pool of fractions from peak 1 (DEAE-650M column).
   Lane 5: Pool of fractions from peak 2 (DEAE-650M column).
   Lane 6: Pool of fractions from peak 3 (DEAE-650M column).
   Lane 7: Pool of fractions from peaks 4 and 5 (DEAE-650M column).
   Lane 8: Flow through fraction from HAE ceramic type 2 column.
   Lane 9: Pool of fractions from peak 1 (HAE ceramic type 2 column).
   Lane 10: Pool of fractions from peak 2 (HAE ceramic type 2 column).
   Lane 11: Pool of fractions from peak 3 (HAE ceramic type 2 column).
   Lane 12: Pool of fractions from peaks 4 and 5 (HAE ceramic type 2 column).
   Lane 13: Supernatant recovered after dialysis against ammonium sulfate 1.7 M overnight, followed by a centrifugation at 10.000g during 20 minutes.
   Lane 14: Pellet recovered after dialysis against ammonium sulfate 1.7 M overnight, followed by a centrifugation at 10.000g during 30 minutes and resuspended into water.
   Lane 15: Flow through fraction from Butyl HP column.
   Lane 16: Wash fraction from Butyl HP column with 25% of elution buffer.
   Lane 17: Pool of fractions from peak 1 (Butyl HP column).
   Lane 18: Pool of fractions from peak 2 (Butyl HP column).
   Lane 19: Pool of fractions from peak 3 (Butyl HP column).
   Lane 20: Pool of fractions from the superdex 200 prep grade column formulated and concentrated up to 1 mg/ml.
   Lane 21: Benchmark protein ladder.

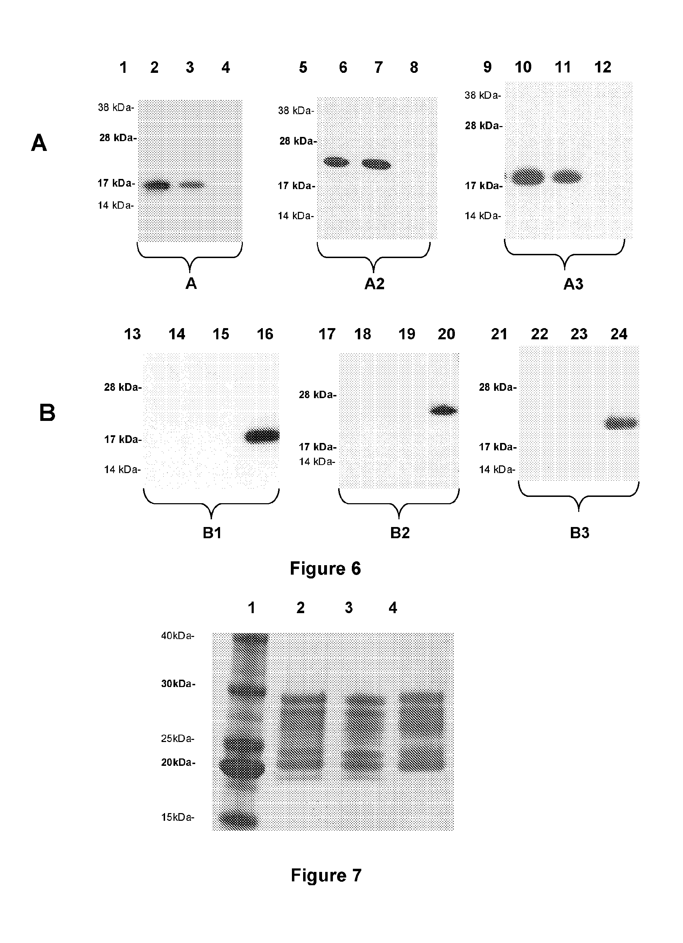

**Figure 6****.** Western blot analyses performed on purified proteasomes with antibodies directed against constitutive or immunoproteasome subunits.
   Lane 1, 5, 9, 13, 17 and 21: SeeBlue® Plus2 Pre-stained Standard.
      A: Antibodies directed against inducible-β subunits
      **A1 - Anti-β1**i
   Lane 2: Purified in-house immunoproteasome expressed from A549 cell line stimulated by IFNγ, transferred onto PVDF membrane and detected with a monoclonal antibody from Enzo life Science directed against the β1i subunit (clone LMP2).
   Lane 3: Purified in-house immunoproteasome expressed from Ramos cell line stimulated by IFNγ, transferred onto PVDF membrane and detected with a monoclonal antibody from Enzo life Science directed against the β1i subunit (clone LMP2).
   Lane 4: Purified in-house constitutive proteasome expressed from A549 cell line, transferred onto PVDF membrane and detected with a monoclonal antibody from Enzo life Science directed against the β1i subunit (clone LMP2).
      **A2- Anti-β2i**
   Lane 6: Purified in-house immunoproteasome expressed from A549 cell line stimulated by IFNγ, transferred onto PVDF membrane and detected with a monoclonal antibody from Santa Cruz directed against the β2i subunit (clone MECL1 C2).
   Lane 7: Purified in-house immunoproteasome expressed from Ramos cell line stimulated by IFNγ, transferred onto PVDF membrane and detected with a monoclonal antibody from Santa Cruz directed against the β2i subunit (clone MECL1 C2).
   Lane 8: Purified in-house constitutive proteasome expressed from A549 cell line, transferred onto PVDF membrane and detected with a monoclonal antibody from Santa Cruz directed against the β2i subunit (clone MECL1 C2).
      **A3 - Anti-β5i**
   Lane 10: Purified in-house immunoproteasome expressed from A549 cell line stimulated by IFNγ, transferred onto PVDF membrane and detected with a monoclonal antibody from Enzo life Science directed against the β5i subunit (clone LMP7-1).
   Lane 11: Purified in-house immunoproteasome expressed from Ramos cell line stimulated by IFNγ, transferred onto PVDF membrane and detected with a monoclonal antibody from Enzo life Science directed against the β5i subunit (clone LMP7-1).
   Lane 12: Purified in-house constitutive proteasome expressed from A549 cell line, transferred onto PVDF membrane and detected with a monoclonal antibody from Enzo life Science directed against the β5i subunit (clone LMP7-1).
      B Antibodies directed against catalytic β subunits
      **B1 - Anti-β1**
   Lane 14: Purified in-house immunoproteasome expressed from A549 cell line stimulated by IFNγ, transferred onto PVDF membrane and detected with a monoclonal antibody from Enzo life Science directed against the β1 subunit (clone MCP 421).
   Lane 15: Purified in-house immunoproteasome expressed from Ramos cell line stimulated by IFNγ, transferred onto PVDF membrane and detected with a monoclonal antibody from Enzo life Science directed against the β1 subunit (clone MCP 421).
   Lane 16: Purified in-house constitutive proteasome expressed from A549 cell line, transferred onto PVDF membrane and detected with a monoclonal antibody from Enzo life Science directed against the β1 subunit (clone MCP 421).
      **B2 - Anti-β2**
   Lane 18: Purified in-house immunoproteasome expressed from A549 cell line stimulated by IFNγ, transferred onto PVDF membrane and detected with a rabbit polyclonal antibody from Boston Biochem directed against the β2 subunit (AP 123).
   Lane 19: Purified in-house immunoproteasome expressed from Ramos cell line stimulated by IFNγ, transferred onto PVDF membrane and detected with a rabbit polyclonal antibody from Boston Biochem directed against the β2 subunit (AP 123).
   Lane 20: Purified in-house constitutive proteasome expressed from A549 cell line, transferred onto PVDF membrane and detected with a rabbit polyclonal antibody from Boston Biochem directed against the β2 subunit (AP 123).
      **B3- Anti-β5**
   Lane 22: Purified in-house immunoproteasome expressed from A549 cell line stimulated by IFNγ, transferred onto PVDF membrane and detected with a rabbit polyclonal antibody from UpState laboratory directed against the β5 subunit (09-278).
   Lane 23: Purified in-house immunoproteasome expressed from Ramos cell line stimulated by IFNγ, transferred onto PVDF membrane and detected with a rabbit polyclonal antibody from UpState laboratory directed against the β5 subunit (09-278).
   Lane 24: Purified in-house constitutive proteasome expressed from A549 cell line, transferred onto PVDF (Polyvinylidene fluoride) membrane and detected with a rabbit polyclonal antibody from UpState laboratory directed against the β5 subunit (09-278).
**Figure 7****.** Quality control by SDS-PAGE in reducing conditions of purified proteasomes expressed in different strains of cell lines stimulated with or without IFNγ.
   Lane 1: Benchmark protein ladder.
   Lane 2: Purified in-house immunoproteasome expressed in A549 cell line stimulated by IFNγ.
   Lane 3: Purified in-house immunoproteasome expressed in Ramos cell line stimulated by IFNγ.
   Lane 4: Purified in-house constitutive proteasome expressed in A549 cell line.

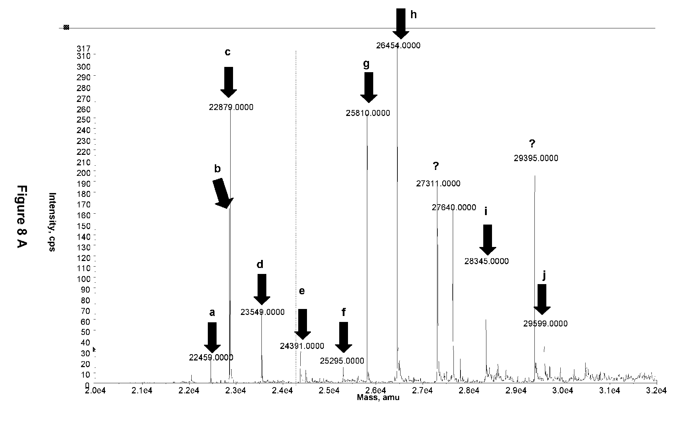

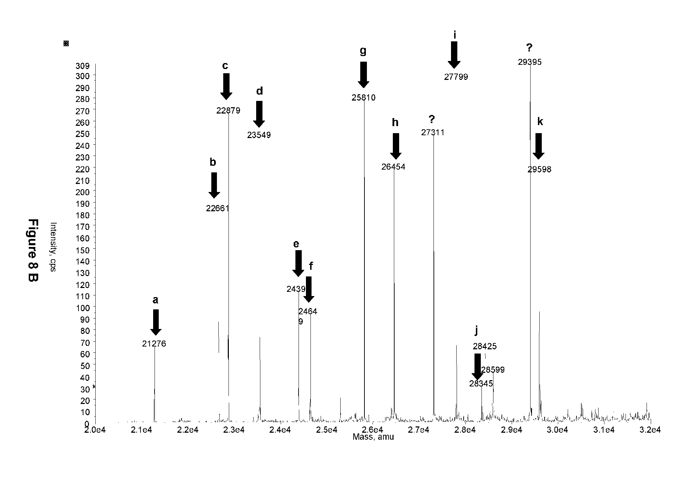

**Figure 8****.** LC-MS analyses of the purified 20S proteasome complexes. The method allows unambiguous identification of all individual subunits.
   **A.** constitutive proteasome expressed in A549 cell line and purified in-house: (a) Ps β type-5; (b) Ps β type-3; (c) Ps β type-2; (d) Ps β type-1; (e) Ps β type-4; (f) Ps β type-7; (g) Ps α type-2; (h) Ps α type-5; (i) Ps α type-3; (j)Ps α type-1; (?) Unknown
   **B.** immunoproteasome expressed in A549 cell line stimulated by IFNγ and purified in-house: (a) Ps β type-1i; (b) Ps β type-5i; (c) Ps β type-2; (d) Ps β type-1; (e) Ps β type-4; (f) Ps β type-2i; (g) Ps α type-2; (h) Ps α type-5; (i) Ps α type-7; (j) Ps α type-3; (k) Ps α type-1; (?) Unknown
**Figure 9****.** Purity assessment of purified immunoproteasome (ip) preparation by quantitative Western blot with antibodies directed against constitutive subunits.
   **Panel A:** identification of the β1 subunit for the constitutive proteasome (cp) with a monoclonal antibody from Enzo life Science (clone MCP 421).
      Lane 1: SeeBlue® Plus2 Pre-stained Standard.
      Lane 2: 6µg of purified immunoproteasome
      Lane 3: 5µg of purified immunoproteasome
      Lane 4: 4µg of purified immunoproteasome
      Lane 5: 3µg of purified immunoproteasome
      Lane 6: 2µg of purified immunoproteasome
      Lane 7: 1µg of purified immunoproteasome
      Lane 8: 0.5µg of purified constitutive proteasome
      Lane 9: 0.25µg of purified constitutive proteasome
      Lane 10: 0.1µg of purified constitutive proteasome
   **Panel B:** identification of the β2 subunit for the constitutive proteasome with a rabbit polyclonal antibody from Boston Biochem (AP 123).
      Lane 11: SeeBlue^{®} Plus2 Pre-stained Standard.
      Lane 12: 6µg of purified immunoproteasome
      Lane 13: 5µg of purified immunoproteasome
      Lane 14: 4µg of purified immunoproteasome
      Lane 15: 3µg of purified immunoproteasome
      Lane 16: 2µg of purified immunoproteasome
      Lane 17: 1µg of purified immunoproteasome
      Lane 18: 0.5µg of purified constitutive proteasome
      Lane 19: 0.25µg of purified constitutive proteasome
      Lane 20: 0.1µg of purified constitutive proteasome
   **Panel C:** identification of the β5 subunit for the constitutive proteasome with a rabbit polyclonal antibody from UpState laboratory (09-278).
      Lane 21: SeeBlue® Plus2 Pre-stained Standard.
      Lane 22: 6µg of purified immunoproteasome
      Lane 23: 5µg of purified immunoproteasome
      Lane 24: 4µg of purified immunoproteasome
      Lane 25: 3µg of purified immunoproteasome
      Lane 26: 2µg of purified immunoproteasome
      Lane 27: 1µg of purified immunoproteasome
      Lane 28: 0.5µg of purified constitutive proteasome
      Lane 29: 0.25µg of purified constitutive proteasome
      Lane 30: 0.1µg of purified constitutive proteasome
**Figure 10****.** Phenyl Sepharose 650M chromatogrpahy.
   Peak A: 1.7M (NH4)₂SO₄ gradient elution. The peak represents contaminants
   Peak B: Wash fraction in which the proteasome is eluted
**Figure 11****.** Western blot analyses performed on purified proteasomes with antibodies directed against (A) immunoproteasome or (B) constitutive subunits.
   Lane 1: SeeBlue® Plus2 Pre-stained Standard.
   Lane 2: Immunoroteasome from Boston Biochem (ref: E-370): 0.5µg loaded onto the gel
   Lane 3: Purified in-house immunoproteasome expressed in A549 cell line stimulated by IFNγ.
   Lane 4: Purified in-house immunoproteasome expressed in Ramos cell line stimulated by IFNγ.
   Lane 5: Constitutive proteasome from Boston Biochem (ref: E-360)
   Lane 6: Purified in-house constitutive proteasome expressed in A549 cell line
**Figure 11** **A:** Western blot with antibodies directed against immunoproteasome subunits
   A1 : Anti-β1i. Mouse Beta 1i subunit, 20S proteasome from Enzo Life sciences (PW8840 batch X07876a).
   A2 Anti-β2i. Anti-Beta 2i subunit, 20S proteasome (mouse monoclonal IgG) clone MECL 1 C2 from Santa Cruz.
   A3: Anti-β5i. Anti-β5i subunit, 20S proteasome (mouse monoclonal IgG) clone LMP7-1 from Enzo life science.
**Figure 11** **B**: Western blot with antibodies directed against constitutive proteasome subunits
   B1 Anti-β1 Anti-20S proteasome subunit Beta 1 (mousse mAb MCP421) from Enzo life sciences.
   B2 Anti-β5: Rabbit Beta 5 subunit, 20S proteasome (UpState, 09-278, batch 0611046110).

### EXAMPLES

### Materials, reagents and methods

### Materials and reagents

Tris (hydroxymethyl) aminomethane, acide 4-(2-hydroxyethyl)-1-piperazine ethane sulfonique (HEPES), sodium chloride (NaCl), dithiothreitol (DTT), glycerol were obtained from Fluka. NaH₂PO₄, Na₂HPO₄, KH₂PO₄, K₂HPO₄, (NH₄)₂SO₄, glycin, Triethanolamine were from Merck chemical. Hi-Load Butyl HP sepharose 16/10, Hi-Load Superdex 200 prepgrade 16/60 chromatographies columns, Gel filtration HMW calibration kit (ref. 28-4038-42), column holders (XK16/20) and the AKTA Explorer 100 equipped with an air detector were from GE-Healthcare Lifesciences. DEAE-50M media was supplied by TosoHaas. CHT Hydroxyapatite ceramic type II resin was purchased from Bio-Rad laboratory. Enzymatic substrates: LLVY-AMC (ref. S4939) and LLE-βNA (ref. C0788) were purchased from Sigma-Aldrich. ARR-AMC peptide was supplied by Calbiochem (ref. 539149). 96 well plates Black Flat Bottom Polystyrene NBS (ref. 3650) were from Corning^{®}. The SYNERGYMx Monochromator-Based Multi-Mode Microplate Reader was from BioTek (Winooski, United States). Concentrators AMICON^{®} Ultracel 30k centrifugal filter devices were supplied by Millipore (UFC 903024).

Antibodies raised against constitutive proteasome or immunoproteasome

Rabbit AP-123 polyclonal antibody raised against β₂-20S human proteasome was purchased from Boston Biochem Inc (Cambridge, United Kingdom). Rabbit 09-278 polyclonal antibody raised against β₅-20S human proteasome from UpState laboratory was supplied by Millipore (Billerica, United States). Monoclonal antibody MCP421 raised against β₁-20S human proteasome, monoclonal antibody LMP2-13 (PW8840) raised against β₁ᵢ-20S human proteasome, monoclonal antibody LMP7-1 (PW8845) raised against β₅ᵢ-20S human proteasome were from Enzo life sciences. Monoclonal antibody MECL 1 C2 (sc-133236) raised against β₂ᵢ-20S human proteasome was purchased from Santa Cruz laboratory. All antibodies were used in western blot at a final dilution of 1:1000 in Phosphate Buffer Saline (PBS) pH 7.3, 0.1% milk except the rabbit AP-123 antibody which was diluted at 1:100 in the same buffer at room temperature.

### Conjugate antibodies

Donkey anti-rabbit HRP (ref. 711-036-152), Donkey anti-mouse HRP (ref. 715-035-151) were purchased from Jackson ImmunoResearch laboratories Inc., and diluted at 1:2000 in 0.1% milk, PBS pH 7.3.

### Cell culture

Two different cell lines expressing human constitutive proteasome were cultivated in the laboratory. The immunoproteasome was produced by stimulation of cells with recombinant human gamma interferon (ᵣIFN-γ). The culture conditions and the operating stimulation are as follows:

### A549 cells

Human alveolar epithelial cell lines A549 (CCL-185™, ATCC) were grown at 37°C, in a humidified atmosphere containing 5% CO₂, at 0.5 to 25 x 10⁴ cells per cm² in DMEM-F12 media supplemented with 10% Ultra Low IgG Fetal Bovine Serum (ref. 16250, Lifetechnologies) and 2 mM of Stable-Glutamine (ref. M11-006, PAA). Cells were cultured using Cellbind Hyperflask M Cell Culture Vessel (ref. 10010, Corning) for 6 days. The attached cells were then harvested using 0.05 % TrypLE™ Express Stable Trypsin-Like Enzyme (ref. 12605-028, Lifetechnologies). The cells were washed with phosphate buffer (PBS) and centrifuged 3 times at 800 x g for 15 min to eliminate the residual trypsin. The pellets were then frozen at -80°C until sufficient material was produced to start the purification process.

### RAMOS B-Cells

Burkitt's lymphoma cell lines Ramos: 2G6.4C10 (CRL-1923™, ATCC) were grown at 37°C, in a humidified atmosphere containing 5% CO₂, at 3 to 30 x 10⁵ cells per milliliter in RPMI 1640 media (ref. 21875-091, Lifetechnologies) supplemented with 10% Ultra Low IgG Fetal Bovine Serum (ref. 16250, Lifetechnologies). The cells were cultured using Cellbind Hyperflask M Cell Culture Vessels (ref. 10010, Corning) for 4 days. A total media exchange was performed on day 3. Static suspension cultures were harvested by centrifugation at 800 x g for 15 min (RC5C centrifuge, Sorvall instruments) and pellets were frozen at -80°C until sufficient material was produced to start the purification process.

### Stimulation of cells by recombinant human gamma interferon

Whatever the stain of cells, the activation was induced by daily stimulation with 100units/ml of ᵣIFN-γ (ref. 300-02, PeproTech EC Ltd.) diluted in culture media.

### Extraction of Proteasome

Frozen pellets of 3.6 x 109 cells (corresponding to a wet weight of 11.35g) were resuspended in the solubilization buffer: 2 mM DTT, Tris-HCl 50 mM pH 7.5 for 10 minutes and sonicated (Inotech, Switzerland) during 2 continuous cycles of 1 min on ice. The suspension was then diluted two times with water and centrifuged at 27,000 x g for 30 minutes at 4°C (RC5C centrifuge, Sorvall instruments). The supernatant was then ultracentrifuged at 70,000 x g for 60 minutes at 4°C (Beckman Optima L-100 equipped with a Ti45 rotor). The supernatant was recovered and 10 % of glycerol was added to the sample. The cell extract was finally filtered on 0.2µm and directly loaded on a DEAE-650M column. All chromatography runs were performed at room temperature.

### Anion exchange chromatography (DEAE-650M)

The cell extract was loaded directly to a XK column (16 by 10 cm) of DEAE-650M (TosoHaas) at 3 ml/min previously equibrated with 3 column volumes (CV) of binding buffer: 1 mM DTT, 25 mM NaCl, 10 % glycerol, 20 mM Triethanolamine pH 7.7. Unbound proteins were removed by washing the column with 2 CV of binding buffer. A first elution step with 12% of elution buffer (1 mM DTT, 10 % glycerol, 500 mM NaCl, 20 mM, triethanolamine pH 7.7) was implemented to remove some contaminants. Then, a gradient from 12 to 60% of elution buffer in 20 CV was performed followed by a sloppy and short gradient from 60 to 100% of elution buffer in 5 CV at a constant flow rate of 3 ml/min. The column was finally washed with 3 CV of elution buffer. Each fraction was tested for its capacity to hydrolyze both peptides: LLVY-AMC (chymotrypsin-like) and LLE-βNa (caspase-like), and fractions containing the major enzymatic activities corresponding to the third peak (between 165 and 265 mM of NaCl) were pooled.

### Mixed mode chromatography (Hydroxyapatite ceramic type II)

The DEAE-650M pool was applied to a XK column (16 by 10 cm) of CHT Hydroxyapatite ceramic 2 (HAE) previously equilibrated with 3 CV at 3 ml/min in 1 mM DTT, 10 % glycerol, 10 mM potassium-phosphate pH 6.8. Adsorbed proteins were eluted with a linear gradient of potassium phosphate at pH 6.8 from 0 to 300 mM at 3 ml/min. Each fraction was tested for its capacity to hydrolyze both peptides: LLVY-AMC (chymotrypsin-like) and LLE-βNa (caspase-like), and fractions containing the major enzymatic activities corresponding to the third peak (between 70 and 106 mM of potassium phosphate) were pooled.

### Dialysis (against ammonium sulfate)

The HAE pool from the previous chromatography was dialyzed against 1.7 M (NH₄)₂SO₄, 50 mM Tris-HCl pH 6.7 overnight with a 30kDa cutoff dialysis membrane. Some precipitation occurred during the night and the precipitate was removed by centrifugation at 10,000 x g for 20 minutes at 4°C. The pellet was discarded and the supernatant directly injected on the hydrophobic interaction chromatography column (HIC).

### Hydrophobic interaction chromatograpjy (Butyl HP sepharose)

The supernatant was applied to a XK column (16 by 10 cm) of Butyl-HP sepharose previously equilibrated with the following binding buffer: 1 mM DTT, 1.7 M **(NH₄)₂SO₄,** 50 mM Tris-HCI pH 6.7 for 3 CV at 3 ml/min. After the loading step, the column was washed with 3 CV of binding buffer and a first step with 20 % of elution buffer: 1 mM DTT, 50 mM Tris-HCI pH 6.7 was carried-out for 3 CV to remove a major contaminant. The adsorbed proteins were then eluted by a decreasing gradient of **(NH₄)₂SO₄** (20 to 80 % of elution buffer) for 20 CV at a constant flow rate of 1 ml/min. Each fraction was tested for its capacity to hydrolyze both peptides: LLVY-AMC (chymotrypsin-like) and LLE-βNa (caspase-like). Three main pools were performed corresponding to the three different peaks observed. Each pool was also tested in western blot to specifically identify the peaks containing the constitutive proteasome or the immunoproteasome species.

### Gel filtration chromatography, concentration and storage

A Hiload 16/60 superdex 200 prep grade column equilibrated with 100 mM NaCl, 1 mM DTT, 50 mM HEPES pH 7.6 was used at 1 ml/min for the polishing step. Different injections were performed whether the interest was for the 20S constitutive proteasome or the immunoproteasome. Concentrated fractions from the Butyl HP chromatography step were applied to the gel filtration column. The fractions which were able to hydrolyze substrates were pooled and concentrated by a 30kDa molecular weight cutoff membrane up to 1 mg/ml. This column was also used to estimate molecular mass of the proteasome complexes. The column was calibrated with the following proteins from the HMW gel filtration kit: Thyroglobulin (669 kDa), Ferritin (449 kDa), Aldolase (158 kDa), Conalbumin (75 kDa) and Ovalbumin (44 kDa). Finally purified 20S constitutive proteasome and immunoproteasome were concentrated around 1 mg/ml in the formulation buffer (100 mM NaCl, 1 mM DTT, 50 mM HEPES pH 7.6) and stored at -80°C.

### Determination of protein concentration and storage

The protein concentration was assessed using the Bio-Rad Protein Assay kit according to the manufacturer's instructions.

### Peptidase activities assays

Peptidase activities of the proteasome were assayed using fluorogenic peptides, N-succinyl-Leu-Leu-Val-Tyr-amidomethylcoumarin (LLVY-AMC) for the chymotrypsin-like activity, benzoyloxycarbonyl-Ala-Arg-Arg-amidomethylcoumarin (ARR-AMC) for the trypsin-like activity, and N-carbobenzoxy-Leu-Leu-Glu-p-naphthylamide (LLE-βNA) for the caspase-like activity. Each chromatography fraction to be tested for hydrolyzing activities was assigned to a 96-well Black Flat Bottom microtiter plate (35 µL). A negative control was also included corresponding to the buffer alone (50 mM Tris-HCI pH 7.5) instead of the sample. 10µL of 5X assay buffer (50 mM MgCl₂, 250 mM Tris-HCI pH 8.3) was added into each well of the microplate and last, 5 µL of the appropriate peptide substrate (LLVY-AMC at 3 mm or LLE-βNA at 5 mm or ARR-AMC at 5 mm) was added at a final volume of 50µl. The plate was then covered and incubated at 37°C over 35 minutes. Enzymatic reactions were conducted in a temperature-controlled microplate fluorimetric. Excitation/emission wavelengths were 370/430 nm, 333/450 nm for aminomethylcoumarin and β-naphthylamine, respectively. The background fluorescence (negative control value) was substracted from each well and fluorescence values were reported on the chromatogram.

### Gel electrophoresis and immunoblotting analysis

All crude extracts and chromatography pools were analyzed by one dimensional SDS-PAGE on NuPage 10% Bis-Tris Gel 1 mm (Invitrogen, NP0302BOX). Proteins were transferred for two hours on a Nitrocellulose membrane (Biorad, 162-01469) previously equilibrated in 380 mM Glycin, 20% methanol, 0.1% SDS, 50mM Tris-HCI pH 7 and analyzed by immunoblotting. For the qualitative western blot study, 0.5µg of proteasome complexes was loaded onto the gels whereas for the quantitative Western blot study, proteasome complexes were overloaded as described in the figure legends.

### Mass Spectrometric Analysis via Liquid Chromatography and Mass Spectrometry (LC-MS)

LC-MS analyses were performed by reversed phase chromatography on a capillary HPLC system (Ultimate 3000, Dionex, Sunnyvale, CA, USA) coupled to a Sciex QSTAR Ultima mass spectrometer (Applied Biosystems, Foster City, CA, USA). A Dionex ProSwift RP-10R monolithic capillary column, 1 mm i.d. x 5cm L. was operated at a flow rate of 30ml/min running a 7 min gradient mix starting with 30% buffer B (0,05% formic acid in acetonitrile) in buffer A (0,05% formic acid in water), ending with 64% buffer B in buffer A. Sample size was one microgram purified proteasome. The mass spectrometer was operated in the positive ion mode between using an electrospray ion source. Mass spectra were recorded between m/z 500 and m/z 2000. A solution of horse heart myoglobin was used as an internal calibrant for each spectrum. The solution was injected into the mass spectrometer using the instrument's syringe pump for 3 min prior to the start of each gradient. The mass spectrometer was operated using the Analyst software and spectra were deconvoluted using Analyst's Biotools.

### Example 1: Purification of the immunoproteasome (A549 and Ramos cell lines)

The A549 cell line was selected due to its facility to express constitutive proteasome. It was then stimulated by γ-IFN to produce immunoproteasomes. Ramos cell line, which belongs to an immune cell line, was also selected to produce immunoproteasomes and was treated like the A549 cells by γ-IFN. The purification process was tested with both cell lines (same protocol). The materials and methods used are described in details in the above "Materials reagents and method" section.

### A549 cell line

The steps of the purification process of the immunoproteasome performed on A549 cell line pre-stimulated by γ-IFN as well as the results obtained are detailed herebelow:

### Proteasome extraction & Anion exchange chromatography

The cultivation of the cells was performed for six days with daily stimulation by γ-IFN. The cells were then harvested and several centrifugation steps later proteasome complexes were extracted. The crude extract was filtered and directly loaded onto the DEAE-650M column pre-equilibrated (Figure 1). The elution profile showed five major peaks. In order to identify with confidence the content of each peak, the fractions were tested for their abilities to hydrolyze peptides corresponding to the chymotrypsin-like or caspase-like substrates. The trypsin-like activity was not screened to identify the peak containing proteasome complexes since the trypsin was used to remove adherent A549 cells from the vessel which would have increased the background signal of the enzymatic reaction. The third peak contained the proteasome complexes since the curves corresponding to the chymotrypsin-like and caspase-like activities were overlapped. The SDS-polyacrylamide gel electrophoresis indicated the presence of proteasome complexes in the third peak where several weak bands ranging from 22 to 30kDa characteristics of the proteasome complexes were identified (lane 6 on figure 5).

### Mixed mode chromatography

The fractions containing proteasome complexes were pooled and directly loaded onto the Hydroxyapatite column (HAE) for the next purification step. The elution was performed by a gradient of potassium phosphate and five major peaks were observed (figure 2). As before, the elution fractions were tested for their abilities to hydrolyze chymotrypsin-like and caspase-like substrates. The results showed that the third elution peak contained major overlapped activities with both chymotrypsin-like and caspase-like signals. The characterization of fractions by SDS-PAGE confirmed the activity results with several bands ranging from 22 to 30kDa characteristics of the proteasome subunits (lane 11 on figure 5). As a consequence, the fractions from the third peak were pooled and dialyzed overnight against a buffer containing 1.7M of ammonium sulfate.

### Hydrophobic interaction chromatography

After centrifugation and filtration, the filtered supernatant that resulted from the precipitation by ammonium sulfate was directly loaded onto a butyl sepharose HP column. Several optimizations were performed to emphasize the separation of proteasome complexes. For instance, a screening experiment of binding buffers to check the appropriate concentration of ammonium sulfate necessary to bind proteasome complexes onto the column was performed. Moreover, the slope of the elution gradient was also optimized. Consequently, three main peaks were eluted from the column (figure 3), with a noteworthy separation and then pooled separately. A first peak was eluted at an average conductivity of 118mS/cm, then a second peak at 112mS/cm and finally a third one at 99mS/cm. The three main peaks showed several proteins bands with molecular weight ranging from 22 to 30kDa (lanes 17, 18 and 19 on figure 5) on SDS-polyacrylamide gel electrophoresis indicating the presence of proteasome complexes in these three peaks. No major difference of electrophoresis mobility was noticed between the three peaks.

In order to confidently identify the three main proteasome complexes, western blot studies were performed to identify which catalytic β-subunits or IFN-γ-inducible β subunits were present in the samples. Each elution fractions was submitted to western blot analysis. Different specific antibodies directed against catalytic β-subunits or inducible-β subunits by γ-IFN were used for the identification of proteasome complex species (figure 4). The Fractions from C6 to D11, which composed the first elution peak, were all recognized by the anti-β1i, anti-β2i and anti-β5i antibodies. However they were not recognized by the anti-β1, anti-β2 and anti-β5 antibodies, which detect the constitutive proteasome. These results proved that the first elution peak of the butyl HP sepharose column could be assigned to the immunoproteasome and was not contaminated by constitutive proteasome. The second peak eluted at an average conductivity of 112 mS/cm was also tested in western blot to safely identify which forms of proteasome were present.

The western blot results were more complex to interpret. Actually, the peak contained hybrid proteasomes composed of IFN-γ-inducible β subunits and catalytic β subunits since antibodies directed against β1i, β5i, β1 and β5 were all reactive towards the fractions. This peak was called "intermediate immunoproteasome". An intermediate immunoproteasome is composed of one or two constitutive β subunits. Finally, the third peak characterized by an average elution of 99mS/cm was accurately identified as the constitutive proteasome, because fractions that composed this peak were only recognized by antibodies directed against the 13 catalytic subunits of the constitutive proteasome.

Moreover, A549 cells non- treated with IFN-γ were also purified following this procedure. A single peak characterized by the same conductivity (99mS/cm) was observed. Thus, the constitutive proteasome was not contaminated by immunoproteasome. Regarding the capacity of the samples to hydrolyze synthetic peptides, several differences were observed between the three main species present (figure 3). The major signal for the chymotrypsin-like activity was encountered in the first peak, whereas the major signal for the caspase-like activity was found in the second peak. Interestingly, the absorbance at 280nm was maximal in the first peak. The ratios for chymotrypsin-like versus caspase-like activities were significantly different for the first two peaks containing immunoproteasome species, showing that their subunits contents were probably different confirming Boes et al. (1994) findings that IFN-γ stimulation induces a change in the proteasome subunit composition. The results of this separation step demonstrated that catalytic β subunits activated or not by γ-IFN significantly changd the hydrophobic properties of proteasome complexes. The homogenous immunoproteasome exclusively composed of inducible-β subunits was less hydrophobic than the constitutive proteasome. Such separation was sensitized enough to make the difference between the "intermediate immunoproteasome" composed of a mix of β inducible or catalytic subunits and the immunoprotasome containing only IFN-γ-inducible β subunits.

### Size exclusion chromatography

The first peak eluted on the butyl HP column was pooled and injected onto a size exclusion chromatography for the polishing step in order to exchange the formulation buffer. The peak eluted from the column was symmetric and the molecular weight of the complex was estimated to be 700kDa by gel filtration. The fractions containing the immunoproteasome were pooled and the purity of the pool was controlled by SDS-PAGE (lane 20, figure 6). The profile on SDS-PAGE showed several protein bands corresponding to the subunits of the immunoproteasome without any contamination from host cell proteins. This chromatography step was not necessary to polish the immunoproteasome fraction since the material eluted from the butyl HP column was already pure. Nonetheless, the size exclusion chromatography step was kept in the purification sequence because this column was quite useful for the purification of the 20S constitutive proteasome. In fact, the constitutive proteasome was co-eluted on the butyl HP sepharose column with a contaminant of high molecular weight which is easily removed by gel filtration (data not shown).

### Ramos cell line

The purification process described above for the A549 cell line was applied to the Ramos cell line and the results are as follows:
Table 1 below, shows the results of the purification of immunoproteasome from Ramos cell line stimulated by IFNγ. The chymotrypsin-like activity was measured with LLVY-AMC peptide as the substrate; the caspase-like activity was measured with LLE-βNa peptide as the substrate and the trypsin-like activity with ARR-AMC peptide as the substrate. Specific activity corresponds to the total enzymatic activity divided by the total protein content.

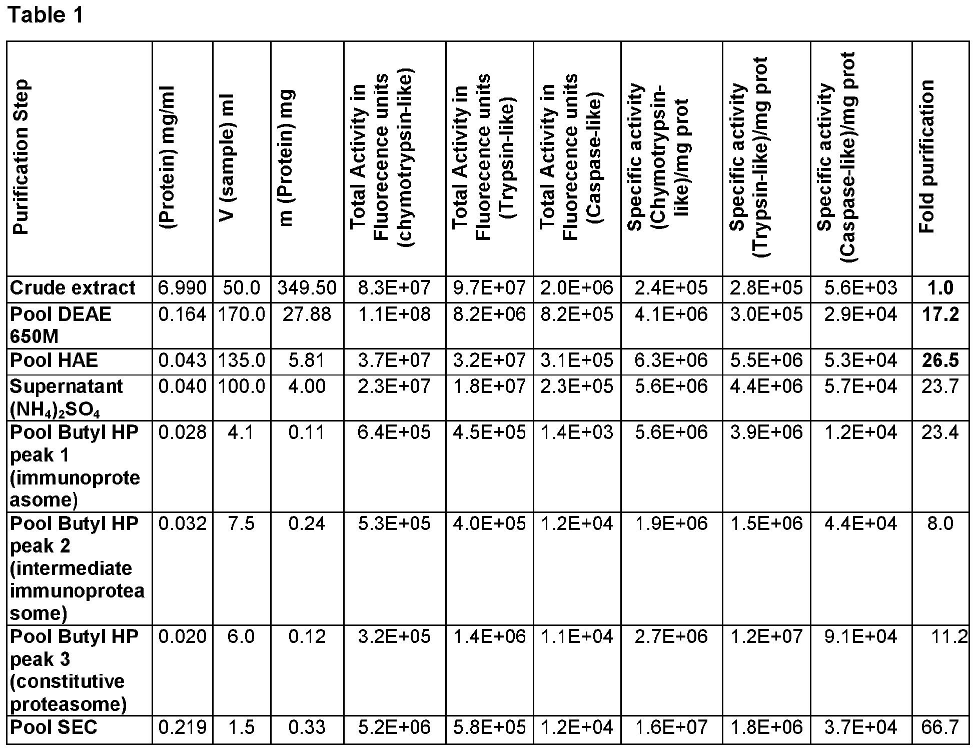

As shown in table 1, the four-step purification procedure led to a proteolytically active purified immunoproteasome. The chymotrypsin-like, caspase-like and also trypsin-like activities were all present in the final purified sample. The immunoproteasome was 66.7 fold purified when compared to the crude extract. The enrichment of the three specific activities was measured all over the purification process and obviously increased with the purity.

### Example 2: Process applied to several batches

In order to make sure that this the protocol was reliable, several batches of constitutive proteasomes and immunoproteasomes expressed from different cell lines were produced with the protocol described above. Purified proteasome complexes were controlled by western blot analysis (figure 6). All immunoproteasome preparations from A549 or Ramos cell lines were easily recognized by antibodies directed against inducible-β subunits (figure 6 panel A) whereas there were not recognized by antibodies directed against catalytic β subunits (figure 6 panel B). However, constitutive proteasomes produced from A549 cell line reacted strongly with antibodies directed against catalytic β subunits and did not react at all with antibodies directed against inducible-β subunits.

Several batches of purified proteasomes expressed in different cell lines were also compared by SDS-PAGE (figure 7). The results showed that whatever the cell line used (A549 or Ramos cells) to express and purify the immunoproteasomes, no difference of pattern was noted. Different bands more or less intensive with molecular masses ranging from approximately 20 to 32kDa were present corresponding to the different α and β subunits. The purified constitutive proteasomes were also compared to the immunoproteasomes and the SDS-PAGE pattern was slightly different. Two main differences were observed. A band ranging around 18kDa was observed in the immunoproteasome preparation whereas it was not present in the constitutive one. Additionally, a band characterized by a molecular weight around 25kDa was observed in both immunopreparations and not in the constitutive one. Actually, these bands are not identified so far. These results showed that constitutive proteasomes and immunoproteasomes have different mobility profiles on SDS-PAGE. Moreover, whatever the cell line used (A549 or Ramos B cells) to produce the immunoproteasomes, the purification process described in this application is able to provide pure material.

### Example 3: Liquid Chromatography-tandem Mass Spectrometry

Recently, Liquid Chromatography-tandem Mass Spectrometry (LC-MS/MS) was employed in elucidating the proteomic profile of the purified human 26S proteasome to further understanding this degradation machinery. The complete composition of the 26S complex, including recently assigned new subunits, was analysed by LC-MS/MS. Aside from the subunit composition, the N-terminal modification and phosphorylation of the proteasome subunits have been characterized by Wang and al (2007); the results from their study are summarized in table 2.

**Table 2. Theoretical masses expected for the constitutive proteasome and immunoproteasome subunits and identification of the Post Translational Modification (PTM).**

| **Proteasome subunit** | **Theoretical Molecular Weight (Da)** | **Expected Molecular Weight (Da)** | **N-terminal Post translational Modification** |
|---|---|---|---|
| **α-1** | 29556 | 29598 | N-acetylmethionine |
| **α-2** | 25767.4 | 25810 | N-acetylalanine |
| **α-3** | 28302.1 | 28344 | N-acetylserine |
| **α-4** | 29483.9 | 29484 | - |
| **α-5** | 26411.1 | 26453 | N-acetylmethionine |
| **α-6** | 27399.5 | 27400 | - |
| **α-7** | 27755.7 | 27798 | N-acetylserine |
| **β-1** | 23549 | 23549 | - |
| **β-2** | 22836.3 | 22878 | N-acetylmethionine |
| **β-3** | 22817.8 | 22860 | N-acetylserine |
| **β-4** | 24391.9 | 24391 | - |
| **β-5** | 22458.4 | 22459 | - |
| **β-6** | 21904 | 21946 | N-acetylalanine |
| **β-7** | 25295.1 | 25295 | - |
| **β-8 (β-5i, LMP7)** | 22659.7 | 22660 | - |
| **β-9 (β-1i, LMP2)** | 21276.1 | 21276 | - |
| **β-10 (β-2i, MECL1)** | 24648.3 | 24648 | - |

Although, the quality control of the preparations was carried out by western blot analysis, it was decided to use the LC-MS analysis as a complementary technique which was faster and consumed less material. For this purpose, purified preparations of constitutive proteasomes and immunoproteasomes were analyzed by LC/MS. After the injection of purified proteasome complexes onto the liquid chromatography, a Total Ion Current (TIC) trace was generated. The reconstructed protein mass peak from the TIC region was created and the molecular weight of 22860 Da identified the proteasome β3 subunit acetylated (+42 Da). Two spectra analysis are shown corresponding respectively to the purified constitutive proteasome (figure 8A) and the purified immunoproteasome (figure 8B).

As expected in both proteasome preparations analyzed, several common α and β subunits were identified in both spectra. The three catalytic subunits β1, β2 and β5 from the constitutive proteasome were clearly identified in the purified constitutive proteasome preparation (figure 8A). The mass accuracies of the three peaks corresponding to the catalytic β subunits were matched perfectly with the theoretical data shown on table 2.

Data from qualitative Western blot experiments combined with the mass spectrometry analysis demonstrated that the purified constitutive preparation was indeed pure and specific. On the other hand, the MS data with the purified immunoproteasome preparation was significantly different (figure 8B). Although, the three IFN-γ-inducible β subunits (β1i/LMP2, β2i/MECL1 and β5i/LMP7) that characterize the immunoproteasome were present in the preparation, two other additional β subunits from the constitutive proteasome (β1, β2) were pointed out. Surprisingly, the results from the western blot study did not show any contamination of this batch with β1 and β2 subunits (figure 6, panel B lanes 14 and 18).

To further estimate the percentage of contamination of the purified immunoproteasome preparation, three quantitative western blot analyses were separately performed with antibodies directed against the three catalytic constitutive subunits (figure 9). For each antibody tested (figure 9 panel A, panel B and panel C), three known concentrations of purified constitutive proteasome were loaded onto the gel and different concentrations of purified immunoproteasomes were tested to assess their content. By comparing the band intensities observed for the immunoproteasome sample with one of the constitutive proteasome reference, it was possible to estimate the ratio of each catalytic subunit (β1, β2 and β5) with IFN-γ-inducible β subunits (β1i/LMP2, β2i/MECL1 and β5i/LMP7) within the purified immunoproteasome sample. The data was synthesized as shown in table 3.

**Table 3: quantification of catalytic β subunits within the purified immunoproteasome preparation by quantitative Western blot analysis.**

| Antibody naming | Minimum amount detected by Western blot | Maximal ratio of constitutive subunits within the purified immunoproteasome preparation |
|---|---|---|
| MAb MCP 421 (anti-β1 subunit) | 0.1µg | β1/β1i ≤ 3.3% |
| MAb AP-123 (anti-β2 subunit) | 0.25µg | β2/β2i ≤ 16.6% |
| MAb Upstate 09-278 (anti-β5 subunit) | 0.05µg | β5/β5i ≤ 1.66% |

The ratio were as follows: β1/β1i ≤ 3.3%, β2/β2i ≤ 16.6% and β5/β5i ≤ 1.66% demonstrating that the immunoproteasome sample contained some intermediate forms that were composed of at least one or two catalytic subunits. Nevertheless, purified immunoproteasome samples were free of constitutive proteasomes because the image on western blot analysis was never positive with the three antibodies (anti-β1, anti-β2 and anti-β5) directed against constitutive subunits.

The presence of catalytic subunits within the immunoproteasome fraction was probably due to the fractions were pooled the during the Butyl sepharose HP chromatography run. Since the second peak corresponding to the "intermediate immunoproteasome" was dragging, some remaining traces were found in the first peak identified as the homogenous immunoproteasome (figure 3). The quantitative western blot study demonstrated that the sensitivities of the antibodies to recognize epitopes were significantly different from one to the other. Although, the monoclonal antibody named AP123 was supposed to be specific against the constitutive β2 subunit, it was not sensitive enough to detect less than 0.25µg of protein (figure 6) which easily explains the reason why no trace of contamination was seen with the qualitative western blot study. Another reason why it was not possible to detect any trace of contamination in the first western blot study could be due to the specificity of antibodies which is not actually proven.

### Example 4: Comparative experiment with the method described by Bauer et al.

Bauer et al (1997) described a method for the purification of proteasomes from a hypothermophilic archaeon (unicellular type of organism) comprising a HIC step on phenyl 650 M. The purification conditions described in the paper, where it is indicated that the proteasome elutes at 0.7 M ammonium sulfate were tested to attempt to separate immunoproteasomes from A549 cells stimulated by IFN-γ.

### Protocol from Bauer et al.

The active fractions containing proteasome species expressed from the Hyperthermophilic Archaeon *Pyrococcus furiosus* cells previously purified by anion exchange chromatography followed by hydroxyapatite chromatography steps were pooled concentrated, equilibrated to buffer A (50 mM Tris/HCI [pH 7.3], 1.7 M ammonium sulfate), and applied to a column (5 by 50 cm) of phenyl Sepharose 650M (Toso-Haas, Montgomeryville, Pa.) which had been previously equilibrated to buffer A. The column was washed with 1,500 ml of buffer A and developed with a 4,500-ml gradient to buffer B (50 mM Tris/HCI, pH 7.3). Proteolytic activity eluted at 0.7M ammonium sulfate and was concentrated, equilibrated to buffer B, and applied to a Pharmacia Sephacryl S300 gel filtration column (V0, 76 ml; total volume [Vt], 200 ml) which had been previously equilibrated with buffer B. Protease activity eluted as a symmetric peak at 87 ml.

### Results

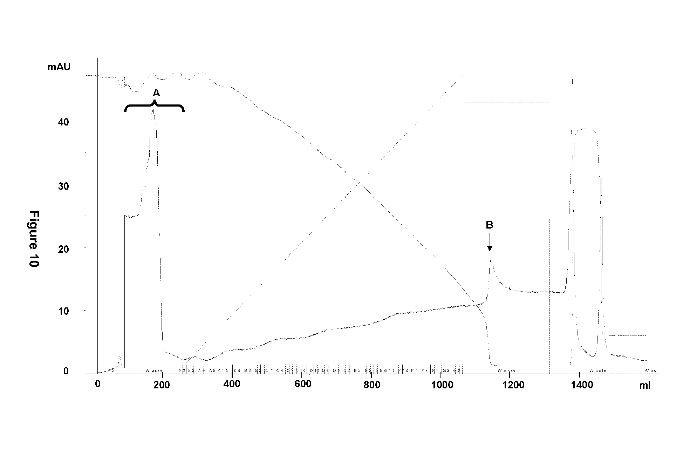

The protocol described above was tested for its efficiency to purify immunoproteasomes expressed from A549 cells stimulated by IFN-γ. The anion exchange chromatography step as well as the hydroxyapatite chromatography step were in accordance with the results obtained by Bauer and al. The Hydrophobic chromatography step was slightly different. After loading the eluate from the Hydroxyapatite column which contained proteasome species, the column was washed with the corresponding buffer used by Bauer and al. A gradient elution from 1.7 to 0.2 M ammonium sulfate was performed after which the column was washed with buffer without ammonium sulfate. No proteasome was eluted from the column during the gradient (peak A in figure 10 represents contaminants), but a peak was detected during the wash with buffer without ammonium sulfate (peak B in figure 10). The Phenyl-650M column used by Bauer and al. revealed that although it was a powerful media for the purification towards host cells proteins, the column was not able to efficiently separate the immunoproteasome from the constitutive proteasome since both proteasome complexes were eluted in the wash fraction.

### Example 5 : Comparative experiment with commercially available iuumoproteasomes

An experiment was performed to compare the cross contamination of a commecially available immunoproteasomes purshased from Boston Biochem (20 S immunoproteasome, human; Cat. # E-370) and the purified immunoproteasome obtained in Example 1. Western blot analyses was performed with antibodies directed against inducible-β subunits (β1i, β2i, β5i) (Figure 11A) and antibodies against catalytic β subunits (β1, β2, β5) (figure 11 B). The following proteasomes complexes were loaded onto the gels and the time exposure with the antibodies was 30 seconds (for details, see the Materials and reagents section of Example 1):
- Immunoproteasome from Boston Biochem (ref: # E-370)
- Purified in-house immunoproteasome expressed in A549 and Ramos cell lines stimulated by IFNγ.
- Constitutive proteasome from Boston Biochem (ref: # E-360)
- Purified in-house constitutive proteasome expressed in A549 cell line

### Results

Figure 11A provides the results obtained with the antibodies directed against inducible-β subunits and shows that the antibodies are working and detecting the subunits.

Figure 11B shows the results obtained with the antibodies directed against constitutive proteasome subunits. The constitutive subunit β1 is detected in the immuno-proteasome (iP) preparation from Boston Biochem but not in the in-house preparations. A trace of the β5 could also be seen (red arrow). β2 was not detected in any of the iP preparations (data not shown). The cross contamination is higher in the commercially available iP in comparison with the iP purified according to the method of the present invention.

### Overall conclusion

The purification method of the invention presents an efficient purification scheme to separate the immunoproteasome from other proteasome complexes. This is the first successful use of HIC chromatography using butyl sepharose column for the separation of immunoproteasomes. The stimulation of cells by γ-IFN induced a change of hydrophobicity in the immunoproteasome leading to a different elution profile on the butyl sepharose column compared to the constitutive proteasome and the intermediate immunoproteasome. The immunoproteasome had a lower hydrophobicity than the constitutive proteasome and eluted in a single peak from the butyl column whereas intermediate forms of the immunoproteasome and the constitutive proteasome were more hydrophobic and as a consequence eluted later from the column.

Additionally, the LC-MS analytical technique method that was implemented has proven to be powerful and effective in verifying the quality of the purified material. This method has also shown to be accurate, reliable and permits unambiguous identification of all individual subunits that compose the 20S proteasome complex. Such technique is able to distinguish the three catalytic β-subunits (β1, β2 and β5) of the constitutive proteasome from the three IFN-γ-inducible catalytic β-subunits (LMP2, MECL-1 and LMP7) of the immunoproteasome. This mass spectrometry analysis is fast, consumes low amount of purified proteasome preparation and can be used as a qualitative method for the evaluation of the immunoproteasome purity. Nonetheless, the quantification of the ratios β1/β1i, β2/β2i and β5/β5i within the immunoproteasome preparation has to be done by quantitative Western blot analysis. Following this improved purification strategy, the material purified was sufficiently pure, specific and fully active in an enzymatic assay performed in vitro.

The combination of the purification method with the characterization by LC-MS provides a powerful and effective tool to generate specific immunoproteasome inhibitors.

### References

1. Akiyama K, Kagawa S, Tamura T, Shimbara N, Takashina M, Kristensen P, Hendil KB, Tanaka K, Ichihara A., Replacement subunits X and Y by LMP7 and LMP2 induced by interferon-gamma for acquirement of the functional diversity responsible for antigen processing, FEBS Lett. 343(1) (1994) 85-8.
2. Bauer MW, Bauer SH, Kelly RM, Purification and Characterization of a Proteasome from the Hyperthermophilic Archaeon Pyrococcus furiosus, Appl Environ Microbiol. 63(3) (1997) 1160-4.
3. Boes B, Hengel H, Ruppert T, Multhaup G, Koszinowski UH, Kloetzel PM, Interferon gamma stimulation modulates the proteolytic activity and cleavage site preference of 20S mouse proteasomes. J Exp Med. 179(3) (1994) 901-9.
4. Bross PF, Kane R, Farrell AT, Abraham S, Benson K, Brower ME, Bradley S, Gobburu JV, Goheer A, Lee SL, Leighton J, Liang CY, Lostritto RT, McGuinn WD, Morse DE, Rahman A, Rosario LA, Verbois SL, Williams G, Wang YC, Pazdur R., Approval summary for bortezomib for injection in the treatment of multiple myeloma. Clin. Cancer Res. 10 (2004) 3954-3964.
5. Brown MG, Monaco JJ., Biochemical purification of distinct protesome subsets, Enzyme Protein. 47 (4-6) (1993) 343-53.
6. Chapiro J, Claverol S, Piette F, Ma W, Stroobant V, Guillaume B, Gairin JE, Morel S, Burlet-Schiltz O, Monsarrat B, Boon T, Van den Eynde BJ., Destructive cleavage of antigenic peptides either by the immunoproteasome or by the standard proteasome results in differential antigen presentation, J. Immunol. 176(2) (2006) 1053-61.
7. Coux O, Tanaka K, Goldberg AL., Structure and functions of the 20S and 26S proteasomes,Annu Rev Biochem. 65 (1996) 801-47.
8. Dahlmann et al. Different proteasome subtypes in a single tissue exhibit different enzymatic properties. J Mol Biol (2000) 303, 643-653.
9. De M, Jayarapu K, Elenich L, Monaco JJ, Colbert RA, Griffin TA, Beta 2 subunit propeptides influence cooperative proteasome assembly, J. Biol. Chem. 278(8) (2003) 6153-9.
10. Demartino GN, Gillette TG, Proteasomes: machines for all reasons, Cell (2007) 129(4) 659-62.
11. Driscoll J, Brown MG, Finley D, Monaco JJ., MHC-linked LMP gene products specifically alter peptidase activities of the proteasome, Nature 365(6443) (1993) 211-2.
12. Eleuteri AM, Angeletti M, Lupidi G, Tacconi R, Bini L, Fioretti E., Isolation and characterization of bovine thymus multicatalytic proteinase complex, Protein Expr Purif. 18(2) (2000) 160-8.
13. Feling RH, Buchanan GO, Mincer TJ, Kauffman CA, Jensen PR, Fenical W., Salinosporamide A: a highly cytotoxic proteasome inhibitor from a novel microbial source, a marine bacterium of the new genus salinospora, Angew Chem Int Ed Engl. 42(3) (2003) 355-7.
14. Gaczynska M, Rock KL, Goldberg AL., Gamma-interferon and expression of MHC genes regulate peptide hydrolysis by proteasomes, Nature (1993) 365(6443) 264-7.
15. Glickman MH, Rubin DM, Coux O, Wefes I, Pfeifer G, Cjeka Z, Baumeister W, Fried VA, Finley D., A subcomplex of the proteasome regulatory particle required for ubiquitin-conjugate degradation and related to the COP9-signalosome and eIF3, Cell. 94(5) (1998) 615-23.
16. Groettrup M, Ruppert T, Kuehn L, Seeger M, Standera S, Koszinowski U, Kloetzel PM., The interferon-gamma-inducible 11 S regulator (PA28) and the LMP2/LMP7 subunits govern the peptide production by the 20 S proteasome in vitro, J. Biol Chem. 270(40) (1995) 23808-15.
17. Groll M, Ditzel L, Löwe J, Stock D, Bochtler M, Bartunik HD, Huber R., Structure of 20S proteasome from yeast at 2.4 A resolution, Nature 386 (6624) (1997) 437-8.
18. Kania MA, Demartino GN, Baumeister W, Goldberg AL, The proteasome subunit, C2, contains an important site for binding of the PA28 (11S) activator, Eur J Biochem. 236(2) (1996) 510-16.
19. Monaco JJ, Nandi D., The genetics of proteasomes and antigen processing, Annu Rev Genet., 29 (1995) 729-54.
20. Nandi D, Marusina K, Monaco JJ., How do endogenous proteins become peptides and reach the endoplasmic reticulum, Curr Top Microbiol Immunol. 232 (1998) 15-47.
21. Orlowski M, Michaud C., Pituitary multicatalytic proteinase complex, Specificity of components and aspects of proteolytic activity, Biochemistry 28(24) (1989) 9270-8.
22. Osmulski PA, Gaczynska M., Atomic force microscopy reveals two conformations of the 20 S proteasome from fission yeast, J Biol Chem. 275(18) (2000) 13171-4.
23. Sacchetta P, Battista P, Santarone S, Di Cola D., Purification of human erythrocyte proteolytic enzyme responsible for degradation of oxidant-damaged hemoglobin, Evidence for identifying as a member of the multicatalytic proteinase family, Biochim Biophys Acta. 1037(3) (1990) 337-43.
24. Saeki Y, Isono E, Toh-E A., Preparation of ubiquitinated substrates by the PY motif-insertion method for monitoring 26S proteasome activity, Methods Enzymol. 399 (2005) 215-27.
25. Sánchez-Serrano I., Success in translational research: lessons from the development of bortezomib, Nat Rev Drug Discov. 5(2) (2006) 107-14.
26. Shah SA, Potter MW, Callery MP., Ubiquitin proteasome pathway: implications and advances in cancer therapy, Surg Oncol. 10(1-2) (2001) 43-52.
27. Tanaka K, Yoshimura T, Kumatori A, Ichihara A, Ikai A, Nishigai M, Kameyama K, Takagi T., Proteasomes (multi-protease complexes) as 20 S ring-shaped particles in a variety of eukaryotic cells, J Biol Chem. 263(31) (1988) 16209-17.
28. Tenzer S, Schild H., Methods Assays of proteasome-dependent cleavage products, Mol Biol. 301 (2005) 97-115.
29. Voges D, Zwickl P, Baumeister W., The 26S proteasome: a molecular machine designed for controlled proteolysis, Annu Rev Biochem, 68 (1999) 1015-68.
30. Wang X, Chen CF, Baker PR, Chen PL, Kaiser P, Huang L., Mass spectrometric characterization of the affinity-purified human 26S proteasome complex, Biochemistry 46(11) (2007) 3553-65.
31. Zwickl P, Kleinz J, Baumeister W., Critical elements in proteasome assembly, Nat Struct Biol. 1(11) (1994) 765-70.

## Claims

1. A method for the purification of immunoproteasomes comprising subjecting an immunoproteasome solution to hydrophobic interaction chromatography, wherein the hydrophobic interaction chromatography is performed with a resin comprising butyl residues.

2. The method according to claim 1, wherein the immunoproteasome solution comprises constitutive proteasome or intermediate immunoproteasomes.

3. The method according to any one of claims 1 or 2, wherein prior to hydrophobic interaction chromatography the immunoproteasome solution is subjected to anion exchange chromatography and mixed mode chromatography.

4. The method according to claim 3, wherein the chromatography steps are carried out in the order:
a. Anion exchange chromatography ;
b. Mixed mode chromatography; and
c. Hydrophobic interaction chromatography.

5. The method according to claim 1, comprising the steps of:
a. subjecting an immunoproteasome solution to anion exchange chromatography to produce a first eluate;
b. subjecting the eluate of step (a) to mixed mode chromatography;
c. subjecting the eluate of step (b) to hydrophobic interaction chromatography wherein the hydrophobic interaction chromatography is performed with a resin comprising butyl residues; and
d. collecting the fraction comprising the immunoproteasome from the eluate of step (c).

6. The method according to claim 6, further comprising a step (e) of size exclusion chromatography.

7. The method according to claim 4 or claim 5, further comprising a dialysis step (b') after the mixed mode chromatography of step (b).

8. The method according to any one of claims 4 to 5, wherein the anion exchange chromatography is performed on a resin comprising diethylaminoethyl (DEAE).

9. The method according to any of claims 4 to 5, wherein the mixed mode chromatography is carried out on a resin comprising ceramic hydroxyapatite.

10. The method according to any preceding claims, wherein the immunoproteasome solution is a fluid derived from a cell culture.

11. The method according to claim 9, wherein the cell culture is yeast or mammalian cell culture.
